# EUROPEAN PATENT APPLICATION

(11) **EP 4 759 724 A1**
(43) Date of publication of application: **17.06.2026**
(21) Application number: 25222543.8
(22) Date of filing: 11.12.2025
(51) Int. Cl.: B64F 5/30, F01D 25/00, C11D 17/00, C12N 9/00

(54) **WASH SYSTEM FOR GAS TURBINE ENGINES**

(30) Priority: 13.12.2024 IN 202411098695; 03.07.2025 US 202519259100
(71) Applicant: General Electric Company, Cincinnati, Ohio 45215 (US)
(72) Inventor: PETKAR, Kirti Arvind, 560066 Bengaluru (IN); GANIGER, Ravindra Shankar, 560066 Bengaluru (IN); YAMARTHI, David Raju, 560066 Bengaluru (IN); MATHEW, Paul, 560066 Bengaluru (IN); MORRA, Martin Matthew, 12345 Schenectady (US); SHETTY, Anil, 560066 Bengaluru (IN)
(74) Representative: Openshaw & Co.

(57) **Abstract**

A wash system (300) for washing a gas turbine engine (10) having a compressor section (22, 24), a combustion section (26), and a turbine section (28, 30) includes a detergent reservoir (365) for storing a wash fluid (372), a foam wash module (315) having a foam generating device in fluid communication with the detergent reservoir (365) and configured for generating a flow of foamed wash fluid (376), one or more additive reservoirs (370) for storing at least one additive material (380), and at least one supply line (378) in communication with the foam wash module (315) and the one or more additive reservoirs (370). The at least one additive material (380) includes a self-propelling material, an exothermic material, or both the self-propelling material and the exothermic material. The at least one supply line (378) supplies the foamed wash fluid (376) and the at least one additive material (380) to the gas turbine engine (10).

## Description

### CROSS-REFERENCE TO RELATED APPLIATION(S)

This application claims priority to Indian Provisional Application No. 202411098695 filed December 13, 2024, and to U.S. Application No. 19/259,100 filed July 3, 2025, which are incorporated herein by reference in their entirety.

### FIELD

The present disclosure relates to a wash system for gas turbine engines, and methods for operating the same.

### BACKGROUND

A gas turbine engine typically includes a fan and a turbomachine. The turbomachine generally includes an inlet, one or more compressors, a combustor, and at least one turbine. The compressors compress air which is channeled to the combustor where it is mixed with fuel. The mixture is then ignited for generating hot combustion gases. The combustion gases are channeled to the turbine(s) which extracts energy from the combustion gases for powering the compressor(s), as well as for producing useful work to propel an aircraft in flight. The turbomachine is mechanically coupled to the fan for driving the fan during operation.

During operation, a substantial amount of air is ingested by such gas turbine engines. Such air may contain foreign particles. While a majority of the foreign particles will follow a gas path through the engine and exit with the exhaust gases, at least a portion of these particles may stick to certain components within the gas turbine engine's gas path, potentially changing aerodynamic and/or thermal properties of the engine and reducing engine performance.

In order to remove such foreign particles from within the gas path of the gas turbine engine, a cleaning operation can be performed that directs water or other fluids towards an inlet of the gas turbine engine. However, such cleaning operations may require elongated soaking times. Additionally, a temperature of the water or other fluids may fall below a desired temperature for cleaning.

Accordingly, wash systems for providing improved cleaning of a gas turbine engine would be useful. More particularly, wash systems that reduce overall cleaning time, maintain desired temperatures, and improve cleaning efficiency are desirable.

### BRIEF DESCRIPTION OF THE DRAWINGS

A full and enabling disclosure of the present disclosure, including the best mode thereof, directed to one of ordinary skill in the art, is set forth in the specification, which makes reference to the appended figures, in which:
FIG. 1 is a cross-sectional view of a gas turbine engine in accordance with an exemplary aspect of the present disclosure.
FIG. 2 is a perspective view of a modular cart that may contain certain components of an exemplary wash system in accordance with an exemplary aspect of the present disclosure.
FIG. 3 is a schematic view of the exemplary wash system of FIG. 2 in accordance with an exemplary aspect of the present disclosure.
FIG. 4 is a perspective view of a microtubule of an additive material for the wash system in accordance with an exemplary aspect of the present disclosure.
FIG. 5A is a schematic diagram of a first flow characteristic of a wash fluid of the wash system in accordance with an exemplary aspect of the present disclosure.
FIG. 5B is a schematic diagram of a second flow characteristic of a wash fluid of the wash system in accordance with an exemplary aspect of the present disclosure.
FIG. 6 is a schematic diagram of the wash system of FIG. 3 operable with the gas turbine engine in accordance with an exemplary aspect of the present disclosure.
FIG. 7 provides a method of cleaning a gas turbine engine in accordance with an exemplary aspect of the present disclosure.

### DETAILED DESCRIPTION

Reference will now be made in detail to present embodiments of the disclosure, one or more examples of which are illustrated in the accompanying drawings. The detailed description uses numerical and letter designations to refer to features in the drawings. Like or similar designations in the drawings and description have been used to refer to like or similar parts of the disclosure.

The word "exemplary" is used herein to mean "serving as an example, instance, or illustration." Any implementation described herein as "exemplary" is not necessarily to be construed as preferred or advantageous over other implementations. Additionally, unless specifically identified otherwise, all embodiments described herein should be considered exemplary.

The singular forms "a," "an," and "the" include plural references unless the context clearly dictates otherwise.

The term "at least one of" in the context of, e.g., "at least one of A, B, and C" refers to only A, only B, only C, or any combination of A, B, and C.

The term "turbomachine" refers to a machine including one or more compressors, a heat generating section (e.g., a combustion section), and one or more turbines that together generate a torque output.

The term "gas turbine engine" refers to an engine having a turbomachine as all or a portion of its power source. Example gas turbine engines include turbofan engines, turboprop engines, turbojet engines, turboshaft engines, etc., as well as hybrid-electric versions of one or more of these engines.

The term "combustion section" refers to any heat addition system for a turbomachine. For example, the term combustion section may refer to a section including one or more of a deflagrative combustion assembly, a rotating detonation combustion assembly, a pulse detonation combustion assembly, or other appropriate heat addition assembly. In certain example embodiments, the combustion section may include an annular combustor, a can combustor, a cannular combustor, a trapped vortex combustor (TVC), or other appropriate combustion system, or combinations thereof.

The terms "low" and "high," or their respective comparative degrees (e.g., -er, where applicable), when used with a compressor, a turbine, a shaft, or spool components, etc. each refer to relative speeds within an engine unless otherwise specified. For example, a "low turbine" or "low speed turbine" defines a component configured to operate at a rotational speed, such as a maximum allowable rotational speed, lower than a "high turbine" or "high speed turbine" of the engine.

The terms "forward" and "aft" refer to relative positions within a gas turbine engine or vehicle, and are based on a normal operational attitude of the gas turbine engine or vehicle. More particularly, forward and aft are used herein with reference to a direction of travel of the vehicle and a direction of propulsive thrust of the gas turbine engine.

The terms "upstream" and "downstream" refer to the relative direction with respect to fluid flow in a fluid pathway. For example, "upstream" refers to the direction from which the fluid flows, and "downstream" refers to the direction to which the fluid flows.

As used herein, the terms "axial" and "axially" refer to directions and orientations that extend substantially parallel to a centerline of the gas turbine engine. Moreover, the terms "radial" and "radially" refer to directions and orientations that extend substantially perpendicular to the centerline of the gas turbine engine. In addition, as used herein, the terms "circumferential" and "circumferentially" refer to directions and orientations that extend arcuately about the centerline of the gas turbine engine.

As used herein, a "bypass ratio" of a turbine engine is a ratio of bypass air through a bypass of the turbine engine to core air through a core inlet of a turbomachine of the turbine engine. For example, the bypass ratio is a ratio of bypass air entering the bypass airflow passage to core air entering the turbomachine.

The terms "coupled," "fixed," "attached to," and the like refer to both direct coupling, fixing, or attaching, as well as indirect coupling, fixing, or attaching through one or more intermediate components or features, unless otherwise specified herein.

As used herein, the terms "first," "second," and "third" may be used interchangeably to distinguish one component from another and are not intended to signify location or importance of the individual components.

For purposes of the description hereinafter, the terms "upper," "lower," "right," "left," "vertical," "horizontal," "top," "bottom," "lateral," "longitudinal," and derivatives thereof shall relate to the embodiments as they are oriented in the drawing figures. However, it is to be understood that the embodiments may assume various alternative variations, except where expressly specified to the contrary. It is also to be understood that the specific devices illustrated in the attached drawings, and described in the following specification, are simply exemplary embodiments of the disclosure. Hence, specific dimensions and other physical characteristics related to the embodiments disclosed herein are not to be considered as limiting.

The present disclosure is generally related to wash systems for removing foreign particles from gas turbine engines. Traditional wash systems require long soaking times. During such time, a temperature of wash fluid for cleaning one or more components of the gas turbine engine may fall below a desired temperature. In an example embodiment, the present disclosure is directed to introducing additives that release heat and increase temperature locally such that a desired temperature of the wash fluid may be maintained. Additionally, or alternatively, the additives of the present disclosure include self-propelling materials for circulating and propelling the wash fluid along surfaces of the gas turbine engine with little to no external effort. The self-propelling materials enable the wash fluid to reach recesses, crevices, occluded surfaces, and/or other hard to reach regions of the gas turbine engine to improve cleaning efficiency.

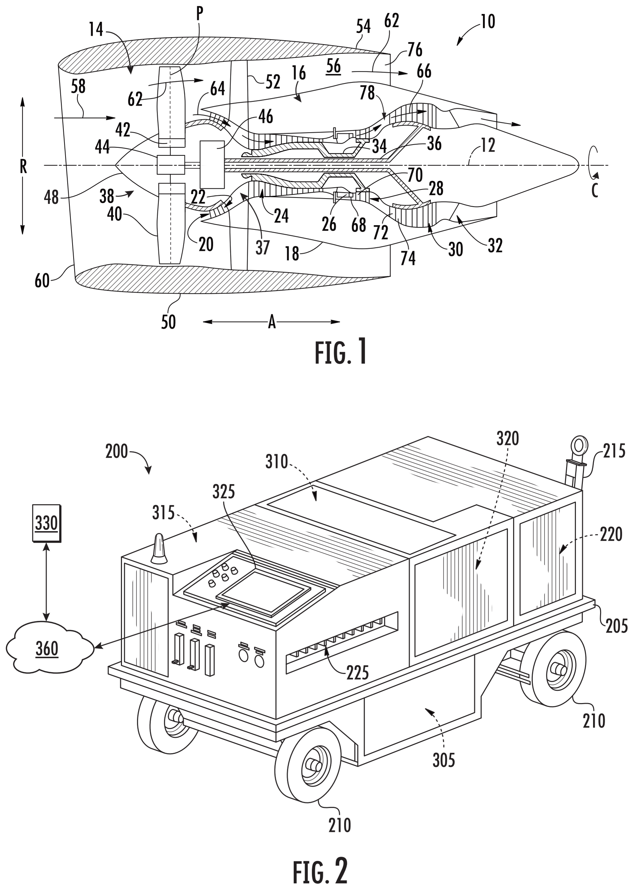

Referring now to the drawings, FIG. 1 is a schematic cross-sectional view of a gas turbine engine 10 in accordance with an exemplary embodiment of the present disclosure. More particularly, for the embodiment of FIG. 1, the gas turbine engine 10 is a high-bypass turbofan jet engine, sometimes also referred to as a "turbofan engine." As shown in FIG. 1, the gas turbine engine 10 defines an axial direction A (extending parallel to a longitudinal centerline 12 provided for reference), a radial direction R, and a circumferential direction C extending about the longitudinal centerline 12. In general, the gas turbine engine 10 includes a fan section 14 and a turbomachine 16 disposed downstream from the fan section 14.

The exemplary turbomachine 16 depicted generally includes a substantially tubular outer casing 18 that defines an annular inlet 20. The outer casing 18 encases, in serial flow relationship, a compressor section including a booster or low pressure (LP) compressor 22 and a high pressure (HP) compressor 24; a combustion section 26; a turbine section including a high pressure (HP) turbine 28 and a low pressure (LP) turbine 30; and a jet exhaust nozzle section 32. A high pressure (HP) shaft 34

(which may additionally or alternatively be a spool) drivingly connects the HP turbine 28 to the HP compressor 24. A low pressure (LP) shaft 36 (which may additionally or alternatively be a spool) drivingly connects the LP turbine 30 to the LP compressor 22. The compressor section, combustion section 26, turbine section, and jet exhaust nozzle section 32 together define a core gas flowpath 37.

For the embodiment depicted, the fan section 14 includes a fan 38 having a plurality of fan blades 40 coupled to a disk 42 in a spaced apart manner. As depicted, the fan blades 40 extend outwardly from disk 42 generally along the radial direction R. Each fan blade 40 is rotatable relative to the disk 42 about a pitch axis P by virtue of the fan blades 40 being operatively coupled to a suitable pitch change mechanism 44 configured to collectively vary the pitch of the fan blades 40, e.g., in unison. The gas turbine engine 10 further includes a power gear box 46, and the fan blades 40, disk 42, and pitch change mechanism 44 are rotatable together about the longitudinal centerline 12 by LP shaft 36 across the power gear box 46. The power gear box 46 includes a plurality of gears for adjusting a rotational speed of the fan 38 relative to a rotational speed of the LP shaft 36, such that the fan 38 may rotate at a more efficient fan speed.

Referring still to the exemplary embodiment of FIG. 1, the disk 42 is covered by rotatable front hub 48 of the fan section 14 (sometimes also referred to as a "spinner"). The front hub 48 aerodynamically contoured to promote an airflow through the plurality of fan blades 40.

Additionally, the exemplary fan section 14 includes an annular fan casing or outer nacelle 50 that circumferentially surrounds the fan 38 and/or at least a portion of the turbomachine 16. It should be appreciated that the nacelle 50 is supported relative to the turbomachine 16 by a plurality of circumferentially-spaced outlet guide vanes 52 in the embodiment depicted. Moreover, a downstream section 54 of the nacelle 50 extends over an outer portion of the turbomachine 16 so as to define a bypass airflow passage 56 therebetween.

During operation of the gas turbine engine 10, a volume of air 58 enters the gas turbine engine 10 through an associated inlet 60 of the nacelle 50 and fan section 14. As the volume of air 58 passes across the fan blades 40, a first portion of air 62 is directed or routed into the bypass airflow passage 56 and a second portion of air 64 as indicated by arrow 64 is directed or routed into the core gas flowpath 37, or more specifically into the LP compressor 22. The ratio between the first portion of air 62 and the second portion of air 64 is commonly known as a bypass ratio. A pressure of the second portion of air 64 is then increased as it is routed through the HP compressor 24 and into the combustion section 26, where it is mixed with fuel and burned to provide combustion gases 66.

The combustion gases 66 are routed through the HP turbine 28 where a portion of thermal and/or kinetic energy from the combustion gases 66 is extracted via sequential stages of HP turbine stator vanes 68 that are coupled to the outer casing 18 and HP turbine rotor blades 70 that are coupled to the HP shaft 34, thus causing the HP shaft 34 to rotate, thereby supporting operation of the HP compressor 24. The combustion gases 66 are then routed through the LP turbine 30 where a second portion of thermal and kinetic energy is extracted from the combustion gases 66 via sequential stages of LP turbine stator vanes 72 that are coupled to the outer casing 18 and LP turbine rotor blades 74 that are coupled to the LP shaft 36, thus causing the LP shaft 36 to rotate, thereby supporting operation of the LP compressor 22 and/or rotation of the fan 38.

The combustion gases 66 are subsequently routed through the jet exhaust nozzle section 32 of the turbomachine 16 to provide propulsive thrust. Simultaneously, the pressure of the first portion of air 62 is substantially increased as the first portion of air 62 is routed through the bypass airflow passage 56 before it is exhausted from a fan nozzle exhaust section 76 of the gas turbine engine 10, also providing propulsive thrust. The HP turbine 28, the LP turbine 30, and the jet exhaust nozzle section 32 at least partially define a hot gas path 78 for routing the combustion gases 66 through the turbomachine 16.

It should be appreciated, however, that the exemplary gas turbine engine 10 depicted in FIG. 1 is by way of example only, and that in other exemplary embodiments, the gas turbine engine 10 may have any other suitable configuration. For example, although the gas turbine engine 10 depicted is configured as a ducted gas turbine engine (i.e., including the outer nacelle 50), in other embodiments, the gas turbine engine 10 may be an unducted gas turbine engine (such that the fan 38 is an unducted fan, and the outlet guide vanes 52 are cantilevered from the outer casing 18). Additionally, or alternatively, although the gas turbine engine 10 depicted is configured as a geared gas turbine engine (i.e., including the power gear box 46) and a variable pitch gas turbine engine (i.e., including a fan 38 configured as a variable pitch fan), in other embodiments, the gas turbine engine 10 may additionally or alternatively be configured as a direct drive gas turbine engine (such that the LP shaft 36 rotates at the same speed as the fan 38), as a fixed pitch gas turbine engine (such that the fan 38 includes fan blades 40 that are not rotatable about a pitch axis P), or both. It should also be appreciated, that in still other exemplary embodiments, aspects of the present disclosure may be incorporated into any other suitable gas turbine engine. For example, in other exemplary embodiments, aspects of the present disclosure may (as appropriate) be incorporated into, e.g., a turboprop gas turbine engine, a turboshaft gas turbine engine, or a turbojet gas turbine engine.

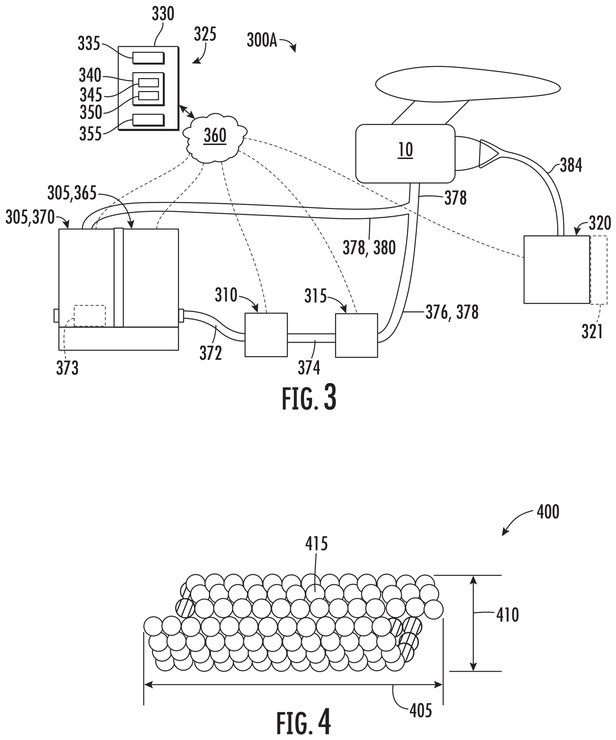

FIG. 2 is a perspective view of a modular cart 200 that may contain certain components of an exemplary wash system in accordance with an exemplary aspect of the present disclosure. As described in detail below, the modular cart 200 may be configured for housing some or all components of a wash system (which will be described in more detail below with reference to FIG.3). The wash system is generally configured for washing, rinsing, or otherwise cleaning a gas turbine engine, such as the gas turbine engine 10 (FIG. 1). Additionally, or alternatively, however, the wash system may be utilized with any other suitable gas turbine engine, such as a turbofan engine, turboprop engine, a turboshaft engine, turbojet engine, etc.

According to the exemplary illustrated embodiment, the wash system is configured as a modular system that is housed at least in part on or within the modular cart 200. As illustrated, the modular cart 200 includes a support frame 205 mounted on a plurality of wheels 210 to improve the mobility of the cart and facilitate quick and easy cleaning of gas turbine engine 10. In addition, the modular cart 200 may include a pivoting tug bar 215 such that the modular cart 200 may be towed by a vehicle to a desired location proximate to the gas turbine engine 10. Moreover, the modular cart 200 can contain various storage compartments 220 for storing all equipment necessary for cleaning the gas turbine engine 10 and other features for facilitating the quick and easy cleaning of the gas turbine engine 10. The modular cart 200 may also include a distribution manifold 225 configured to be fluidly coupled to one or more supply lines for supplying wash fluid and other cleaning materials from the various storage compartments 220 of the modular cart to the gas turbine engine 10.

FIG. 3 is a schematic view of a wash system 300 in accordance with an exemplary aspect of the present disclosure. More specifically, FIG. 3 provides a schematic view of the wash system 300 and its various cleaning modules in accordance with an exemplary embodiment of the present disclosure. For example, the wash system 300 generally includes one or more tank modules 305, a wash module 310, a foam wash module 315, and a collection module 320. In general, the tank modules 305 may store wash fluid and other cleaning materials, the wash module 310 may receive and pressurize the wash fluid, the foam wash module 315 may process the wash fluid to form a foamed wash fluid, and the collection module 320 may collects and/or recycle waste wash fluid and foreign materials.

Each of the various modules are, for the embodiment depicted, operably connected to a control system 325. The control system 325 can include one or more computing device(s) 330. The computing device(s) 330 can include one or more processor(s) 335 and one or more memory device(s) 340. The one or more processor(s) 335 can include any suitable processing device, such as a microprocessor, microcontroller, integrated circuit, logic device, and/or other suitable processing device. The one or more memory device(s) 340 can include one or more computer-readable media, including, but not limited to, non-transitory computer-readable media, RAM, ROM, hard drives, flash drives, and/or other memory devices.

The one or more memory device(s) 340 can store information accessible by the one or more processor(s) 335, including computer-readable instructions 345 that can be executed by the one or more processor(s) 335. The instructions 345 can be any set of instructions that when executed by the one or more processor(s) 335, cause the one or more processor(s) 335 to perform operations. In some embodiments, the instructions 345 can be executed by the one or more processor(s) 335 to cause the one or more processor(s) 335 to perform operations, such as any of the operations and functions for which the control system 325 and/or the computing device(s) 330 are configured, the operations for operating the wash system 300 (e.g., method 700), as described herein, and/or any other operations or functions of the one or more computing device(s) 330. The instructions 345 can be software written in any suitable programming language or can be implemented in hardware. Additionally, and/or alternatively, the instructions 345 can be executed in logically and/or virtually separate threads on the one or more processor(s) 335. The one or more memory device(s) 340 can further store data 350 that can be accessed by the one or more processor(s) 335. For example, the data 350 can include data indicative of power flows, data indicative of engine/ aircraft operating conditions, and/or any other data and/or information described herein.

The computing device(s) 330 can also include a communications interface 355 used to communicate, for example, with the other components of the wash system 300. The communications interface 355 can include any suitable components for interfacing with one or more network(s), including for example, transmitters, receivers, ports, controllers, antennas, and/or other suitable components. One or more devices can be configured to receive one or more commands from the computing device(s) 330 or provide one or more commands to the computing device(s) 330.

Moreover, the control system 325 may also be in communication (e.g., via the communications interface 355) with the various modules 305, 310, 315, 320, described below, and may selectively operate the wash system 300 in response to user input and feedback from these modules 305, 310, 315, 320. More specifically, for the embodiment depicted, the control system 325 is configured to communicate through a wireless communication network 360 through communications interface 355, such that the control system 325 may send or receive information and/or commands to or from the various modules 305, 310, 315, 320 of the exemplary wash system 300 wirelessly. It should also be appreciated, however, that in other embodiments, the control system 325 may additionally, or alternatively, use a wired communication bus to communicate with the various modules 305, 310, 315, 320.

The technology discussed herein makes reference to computer-based systems and actions taken by and information sent to and from computer-based systems. One of ordinary skill in the art will recognize that the inherent flexibility of computer-based systems allows for a great variety of possible configurations, combinations, and divisions of tasks and functionality between and among components. For instance, processes discussed herein can be implemented using a single computing device or multiple computing devices working in combination. Databases, memory, instructions, and applications can be implemented on a single system or distributed across multiple systems. Distributed components can operate sequentially or in parallel.

It should be appreciated that the modular cart 200 and the wash system 300 as described herein may allow for a more versatile cleaning system for a gas turbine engine, such as the gas turbine engine 10. For example, utilizing tank modules that are interchangeable may allow for extended washing operations, without having to refill a tank and wait for the wash fluid, if applicable, to heat up to a desired temperature. Instead, once a given tank module has been utilized or depleted by the wash system 300, a second tank module may be connected to the wash system 300 to allow for the washing operations to continue with minimal interruption. Similarly, utilizing a wash module that is interchangeable may allow for multiple wash operations to be completed on a given gas turbine engine without requiring two completely separate wash systems. In addition, according to exemplary embodiments of the present subject matter, a power module could be configured for providing compressed air and/or electrical power, e.g., when the wash system 300 is used in a location where such facilities are inconvenient or unavailable.

It should be appreciated, that as used herein the term "fluid," "wash fluid," "wash liquid," and the like may refer to any suitable fluid for performing washing operations and/or rinsing the gas turbine engine 10. Such wash fluid is typically made up of water that may include other additives such as detergent, soap, or other treatments. For example, the wash fluid may refer to water, or a combination of water and one or more additives such as detergent, soap, corrosion inhibitors, surfactants, bis-tris, and other additives. Moreover, the wash system 300 is not limited to utilizing water or any particular detergent as a wash fluid. Instead, the wash system 300 may utilize any suitable wash liquid for performing desired washing operations of the gas turbine engine.

Still referring to FIG. 3, the tank module 305 may include a plurality of tanks for storing a wash fluid, additives, or other cleaning materials. For example, the tank module 305 may include a detergent reservoir 365 and one or more additive reservoirs 370.

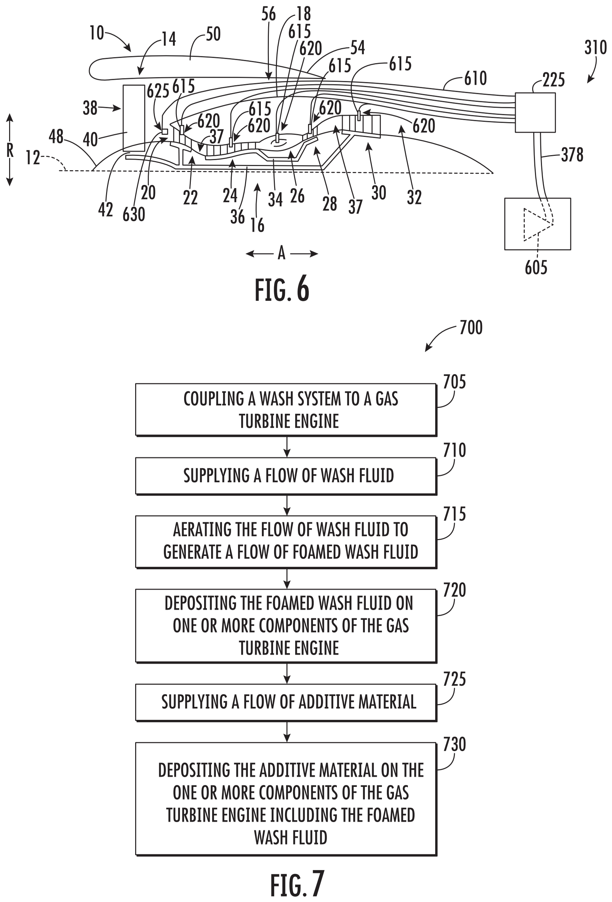

The detergent reservoir 365 contains a wash fluid, or rather a wash liquid. The detergent reservoir 365 is fluidly coupled to a wash module 310. For example, the detergent reservoir 365 may be in fluid communication with the wash module 310 via one or more fluid conduits or tubing. The wash module 310 is configured to receive a flow of the wash fluid 372 from the detergent reservoir 365. For example, the wash module 310 may include a pump (605 in FIG. 6) for receiving the flow of wash fluid 372 and generating a pressurized flow of the wash fluid 374. The control system 325 may be operably coupled to the detergent reservoir 365 and the wash module 310, and more particularly to the pump of the wash module 310, for controlling the flow of wash fluid 372 from the detergent reservoir 365 to the wash module 310.

Additionally, the wash module 310 is in fluid communication with the foam wash module 315. For example, the wash module 310 may be in fluid communication with the foam wash module 315 via one or more fluid conduits or tubing. However, it should be understood that in some embodiments, the wash module 310 and the foam wash module 315 may be integrated into a single, integral module. The foam wash module 315 is configured to receive the flow of wash fluid, such as the pressurized flow of the wash fluid 374, from the wash module 310 and generate a foamed wash fluid. For example, the foam wash module 315 includes a foam generating device for generating a flow of the foamed wash fluid 376.

The foam generating device of the foam wash module 315 may generally be configured for mixing the flow of wash fluid 372 (or the pressurized flow of the wash fluid 374) with a stream of air to aerate the wash fluid and generate the flow of the foamed wash fluid 376. In this regard, for example, the flow of foamed wash fluid 376 may include wash fluid with a desired foam density, or a desired ratio of air to fluid. The foam may be characterized according to different properties as well. For example, the foam generating device may be configured for achieving a specific bubble distribution, foam viscosity, etc. The foam characteristics may be manipulated by adjusting a temperature or a flow rate of either the flow of wash fluid or the stream of air into the foam generating device.

The flow of the foamed wash fluid 376 is supplied from the foam wash module 315 to the gas turbine engine 10 for cleaning by at least one supply line 378. The at least one supply line 378 may include one or more conduits or tubing. Moreover, the control system 325 may be operably coupled to the foam wash module 315 for controlling the flow of the foamed wash fluid 376 to the gas turbine engine 10 via the at least one supply line 378.

The one or more additive reservoirs 370 contains additive material and is in communication with the gas turbine engine 10 via the at least one supply line 378. In at least one example embodiment, an additive pump 373 is in fluid communication with the one or more additive reservoirs 370 for generating a flow of additive material 380. The additive pump 373 may be coupled to or disposed within the one or more additive reservoirs 370. The flow of additive material 380 from the one or more additive reservoirs 370 may be supplied to the gas turbine engine 10 after the flow of foamed wash fluid 376, as will be described below with respect to FIG. 7, such that the additive material does not mix with the foamed wash fluid prior to entering the gas turbine engine 10. In other example embodiments, the flow of additive material 380 and the flow of the foamed wash fluid 376 may be supplied simultaneously and mixed within the at least one supply line 378 upstream of the gas turbine engine 10 to generate a cleaning mixture.

In at least one example embodiment, the additive material stored within the one or more additive reservoirs 370 may include a self-propelling material, an exothermic material, or both the self-propelling material and the exothermic material. For example, the one or more additive reservoirs 370 may include a first additive reservoir for storing the self-propelling material and a second additive reservoir for storing the exothermic material separate from the self-propelling material.

The exothermic material is configured to maintain a desired temperature of the foamed wash fluid 376 within the gas turbine engine 10. For example, the desired temperature of the foamed wash fluid 376 may be greater than or equal to 70°C and less than or equal to 90°C. The exothermic material may provide a sustained exothermic reaction with the foamed wash fluid 376 to maintain the desired temperature. In at least one example embodiment, the exothermic material includes an inert gas. For example, the exothermic material may include nitrogen dioxide (NO2) and dinitrogen tetroxide (N2O4). In such embodiments, an exothermic reaction occurs as 2NO2 dissociates to N2O4, which may increases the temperature of the foamed wash fluid 376.

In additional example embodiments, the exothermic material may include ethylene oxide. The reaction of ethylene oxide with the foamed wash fluid 376 is exothermic such that a temperature of the foamed wash fluid 376 may be increased to maintain the desired temperature of the foamed wash fluid 376.

The self-propelling material is configured to create a swirl that propels the foamed wash fluid 376 along surfaces of the gas turbine engine 10. The active movement of the foamed wash fluid 376 generated by the self-propelling material enables the foamed wash fluid 376 to reach regions of the gas turbine engine 10 that may otherwise be unreachable by the foamed wash fluid 376 alone. For example, the self-propelling material enables the foamed wash fluid 376 to reach recesses, crevices, occluded areas, and small holes, such as cooling holes, for improved cleaning of these regions. Moreover, the self-propelling material may be sized small enough to enter cooling passages of the gas turbine engine 10, which also provides improved cleaning.

In at least one example embodiment, the self-propelling material includes a plurality of microtubules. FIG. 4 illustrates a side view of a microtubule 400 of the plurality of microtubules in accordance with an exemplary aspect of the present disclosure. Each microtubule 400 of the plurality of microtubules may be formed of a plurality of particles 415 including at least one protein and an energy source. For example, the at least one protein may include kinesin and the energy source may include adenosine triphosphate (ATP), gluten, enzymes, or a combination thereof. The kinesin moves along filaments of each of the plurality of microtubules to create active motion of the plurality of microtubules. The energy source, such as ATP, provides power to the at least one protein, such as kinesin. Accordingly, the combination of the at least one protein and the energy source enables active movement of the plurality of microtubules. In at least one example embodiment, each of the plurality of microtubules include greater than or equal to 10% and less than or equal to 20% of the energy source, such as ATP. In at least one example embodiment, each of the plurality of microtubules includes a concentration of ATP greater than or equal to 1 millimolar/liter and less than or equal to 10 millimolar/ liter.

As shown in FIG. 4, each microtubule 400 of the plurality of microtubules may have a cylindrical shape. Additionally, each microtubule 400 may define a length 405 and a width 410 extending perpendicular to the length 405. In at least one example embodiment, the length 405 may be less than or equal to 50 micrometers, and the width 410 may be greater than or equal to 23 nanometers (nm) and less than or equal to 27 nm. Moreover, each microtubule 400 may be hollow and define an inner diameter that is greater than or equal to 11 nm and less than or equal to 15 nm.

In other example embodiments, the self-propelling material may include organic materials, such as proteins and enzymes, cytoplasm found in eukaryotic organisms, and algae. Additionally, the organic material may include dichloromethane or cetyltrimethylammonium bromide (CTAB) aqueous solutions along with the motor proteins like kinesin, myosin, and dynein.

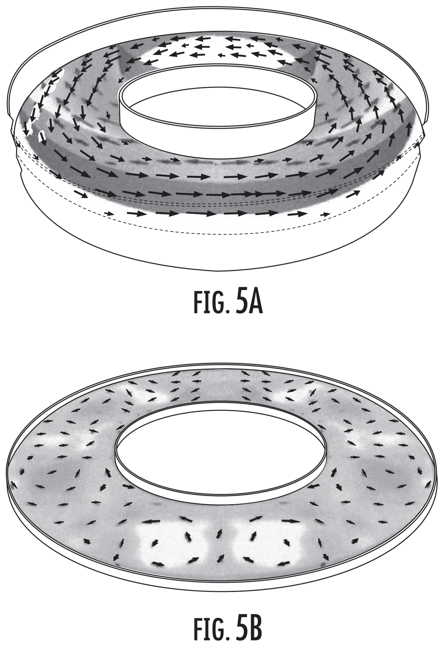

With reference to FIG. 5A illustrating a first flow characteristic of the foamed wash fluid 376 of the wash system 300 and FIG. 5B illustrating a second flow characteristic of the foamed wash fluid 376 of the wash system 300, the plurality of microtubules are configured to create a swirling or vortex effect of the foamed wash fluid 376 within the gas turbine engine 10.

As discussed above, the self-propelling material is configured to create a swirl that propels the foamed wash fluid 376 along surfaces of the gas turbine engine 10. For example, active movement of the plurality of microtubules within the foamed wash fluid 376 generates the swirl. The self-propelling material may generate a constant swirl along surfaces of the gas turbine engine 10, as shown in FIG. 5A, or a more turbulent swirl, as shown in FIG. 5B. The amount of swirl, or vortexing, produced by the self-propelling material may be controlled based on an amount of the self-propelling material injected into the gas turbine engine 10. Additionally, or alternatively, the amount of swirl generated by the self-propelling material may be controlled based on a ratio of an amount of protein and an amount of the energy source included within each microtubule 400 of the plurality of microtubules. In at least one example embodiment, the foamed wash fluid 376 includes greater than or equal to 5% and less than or equal to 25% of the self-propelling material.

Referring again to FIG. 3, in operation, the flow of the foamed wash fluid 376 is supplied to the gas turbine engine 10 via the at least one supply line 378 and deposited on surfaces of one or more components of the gas turbine engine 10. A flow of the additive material 380 is supplied to the gas turbine engine 10 via the at least one supply line 378 after the foamed wash fluid 376 has been deposited on surfaces of the one or more components of the gas turbine engine 10. Alternatively, the flow of the additive material 380 may be supplied simultaneously with the flow of the foamed wash fluid 376 in some example embodiments. The one or more components of the gas turbine engine 10 may include the compressor section (including the LP compressor 22 and the HP compressor 24), the combustion section 26, the turbine section (including the HP turbine 28 and the LP turbine 30), or a combination thereof. Moreover, the one or more components of the gas turbine engine 10 may include one or more nozzles, one or more blades or vanes, a casing, cooling passageways, or other similar components of the gas turbine engine 10.

After washing the gas turbine engine 10, used washing fluid, such as used foamed wash fluid 376, and any excess of the additive material 380 may be discharged from the gas turbine engine 10 to the collection module 320. The collection module 320 may also receive dirt, debris, and other foreign material resulting from cleaning the gas turbine engine 10. As shown in FIG. 3, the dirt, dust, other foreign materials, and any excess foamed wash fluid 376 and additive material 380 may be supplied to the collection module 320 via a collection module supply line 384. The used foamed wash fluid and foreign materials may then be disposed of or recycled from the collection module 320.

In at least one example embodiment, the collection module 320 includes a collection sensor 321. The collection sensor 321 is in fluid communication with the collection module 320 and may be disposed outside or within the collection module 320. The collection sensor 321 is configured to determine a ratio of the additive material 380 to the foamed wash fluid 376 within the used foamed wash fluid received by the collection module 320. In some example embodiments, the collection sensor 321 measures a pH or the total dissolved solids (TDS) of the used foamed wash fluid.

The collection sensor 321 is communicatively coupled to the control system 325 such that the collection sensor 321 may be used to determine a cleaning effectiveness of the gas turbine engine 10 based on the ratio of the additive material 380 to the foamed wash fluid 376 received by the collection module 320. For example, the ratio of additive material 380 to the foamed wash fluid 376 received by the collection module 320 and determined by the sensor may be compared to a desired threshold. Accordingly, a quantity of the additive material 380 supplied to the gas turbine engine 10 may be controlled based on the determined ratio relative to the desired ratio. Moreover, the control system 325 may control delivery of the additive material 380 from the one or more tank modules 305, and more particularly the one or more additive reservoirs 370, to the gas turbine engine 10 based on the ratio of the additive material 380 to the foamed wash fluid 376 determined by the collection sensor 321.

If the determined ratio of the additive material 380 to the foamed wash fluid 376 received by the collection module 320 is greater than the desired threshold, the quantity of the additive material 380 delivered to the gas turbine engine 10 may be reduced or delivery of the additive material 380 may be stopped. If the determined ratio of the additive material 380 to the foamed wash fluid 376 received by the collection module 320 is less than the desired threshold, the quantity of the additive material 380 delivered to the gas turbine engine 10 may be increased. Additionally, if the determined ratio of the additive material 380 to the foamed wash fluid 376 received by the collection module 320 is substantially equal to the desired threshold, the quantity of the additive material 380 delivered to the gas turbine engine 10 may be maintained.

FIG. 6 is a schematic diagram of the wash system 300 of FIG. 3 operable with the gas turbine engine 10 in accordance with an exemplary aspect of the present disclosure. More particularly, FIG. 6 illustrates a schematic view of the wash module 310 of the wash system 300. In certain exemplary embodiments, the wash module 310 of FIG. 6 may be configured in substantially the same manner as exemplary wash module 310 utilized in the exemplary wash system 300 of FIGS. 2-3. For example, the exemplary wash module 310 generally includes a pump 605, the distribution manifold 225 fluidly coupled to the pump 605 for receiving a flow of cleaning material, and a plurality of wash lines 610 fluidly coupled to the distribution manifold 225. The cleaning material may include the flow of the foamed wash fluid 376 and the flow of the additive material 380.

The gas turbine engine 10 defines a plurality of injection ports, such as a plurality of borescope ports 620. For example, as shown in FIG. 6, the turbomachine 16 includes one or more of the plurality of borescope ports 620 defined in the compressor section, in the combustion section 26, and in the turbine section. More specifically, for the embodiment depicted, the turbomachine 16 includes one or more borescope ports 620 defined in the LP compressor 22, the HP compressor 24, a combustion chamber of the combustion section 26, the HP turbine 28, and the LP turbine 30. The borescope ports 620 may allow for inspection of the turbomachine 16 between operations, and more specifically, may open into the core gas flowpath 37 of the gas turbine engine 10 to allow for inspection of, e.g., one or more blades, nozzles, or combustion liners of the gas turbine engine 10 between operations. By contrast, during normal operations, the borescope ports 620 within the combustion section 26 and turbine section may be plugged with a borescope plug (not shown), such that the borescope ports 620 do not affect operation of the gas turbine engine 10.

Moreover, as previously stated, the exemplary gas turbine engine 10 is depicted schematically as being cleaned by the wash module 310 of the wash system 300. In at least one example embodiment, the wash module 310 of the wash system 300 further includes a plurality of spray nozzles 615, with each of the plurality of spray nozzles 615 attached to a respective wash line 610 and configured for extending at least partially into or through one of the borescope ports 620 of the gas turbine engine 10 for providing at least a portion of the flow of the cleaning material, such as the flow of the foamed wash fluid 376 and the additive material 380, to the gas turbine engine 10. It should be appreciated, that in certain example embodiments, the plurality of spray nozzles 615 may extend at least partially into or through borescope ports 620 of the gas turbine engine 10 at locations spaced along, e.g., the circumferential direction C of the gas turbine engine 10. Such a configuration may allow for a more even cleaning of the gas turbine engine 10, including the turbomachine 16, during such wash operations.

The exemplary wash module 310 may further include an inlet nozzle assembly 625 fluidly connected to one or more of the plurality of wash lines 610 for providing at least a portion of the flow of cleaning material to the gas turbine engine 10, or rather to the turbomachine 16, through the inlet 20 of the turbomachine 16. As shown in FIG. 6, the inlet nozzle assembly 625 includes one or more inlet nozzles 630 positioned adjacent the inlet 20 to the turbomachine 16 to inject cleaning material directly into and through the inlet 20 of the turbomachine 16. In other exemplary embodiments, however, the inlet nozzle assembly 625 may instead be located at least partially forward of the fan 38.

Still referring to FIG. 6, the exemplary gas turbine engine 10 includes the outer nacelle 50 which defines the bypass airflow passage 56 with the turbomachine 16. In at least one example embodiment, the plurality of wash lines 610 extend from an aft end of the turbomachine 16, through the bypass airflow passage 56 to each of the respective plurality of borescope ports 620, and to the inlet 20 for the inlet nozzle assembly 625. With such a configuration, the wash system 300 may operate without having to remove one or more portions of the fan section 14. More specifically, a wash system having such a configuration may allow for conducting washing operations (i.e., providing cleaning material through the plurality of wash lines and wash nozzles), while allowing for the gas turbine engine to be cranked or rotated to increase in effectiveness of the washing operations. In addition, a controller, such as the control system 325, may automatically control the speed of the engine core rotation to improve cleaning performance or to prevent unwanted wash fluid intrusion into internal engine air circuits or other passageways.

FIG. 7 provides a method 700 of cleaning the gas turbine engine 10 in accordance with an exemplary aspect of the present disclosure. More specifically, the method 700 of cleaning the gas turbine engine 10 may include cleaning the gas turbine engine 10 with the exemplary wash system 300 discussed with respect to FIGS. 3-6.

In at least one example embodiment, the method 700 includes coupling a wash system to a gas turbine engine at 705, supplying a flow of wash fluid at 710, aerating the flow of wash fluid to generate a flow of foamed wash fluid at 715, depositing the foamed wash fluid on one or more components of the gas turbine engine at 720, supplying a flow of additive material at 725, and depositing the additive material on the one or more components of the gas turbine engine including the foamed wash fluid at 730.

Coupling a wash system to a gas turbine engine at 705 may include fluidly coupling the wash system 300 to the gas turbine engine 10 via the at least one supply line 378. More specifically, the at least one supply line 378 may be fluidly coupled to the plurality of borescope ports 620 of the gas turbine engine 10 via the distribution manifold 225 and wash lines 610, as shown in FIG. 6. Additionally, the modular cart 200 discussed with respect to FIG. 2 may be positioned in proximity to the gas turbine engine 10 for fluidly coupling the at least one supply line 378 of the wash system 300 to the gas turbine engine 10.

Supplying a flow of wash fluid at 710 may include supplying the wash fluid from the detergent reservoir 365 to the wash module 310 and the foam wash module 315. Aerating the flow of wash fluid to generate a flow of foamed wash fluid at 515 may include generating the foamed wash fluid using the foam generating device of the foam wash module 315, as discussed above with respect to FIG. 3.

Depositing the foamed wash fluid on one or more components of the gas turbine engine 10 at 720 includes supplying the flow of the foamed wash fluid 376 to the gas turbine engine 10 via the at least one supply line 378 such that the foamed wash fluid is deposited on surfaces of one or more components of the gas turbine engine 10. For example, the foamed wash fluid 376 may be deposited on surfaces of the compressor section (including the LP compressor 22 and the HP compressor 24), the combustion section 26, the turbine section (including the HP turbine 28 and the LP turbine 30), or a combination thereof.

Supplying a flow of additive material at 725 may include supplying the additive material from the additive reservoir 370 to the gas turbine engine 10 via the at least one supply line 378. Depositing the additive material on the one or more components of the gas turbine engine including the foamed wash fluid at 730 includes injecting the additive material from additive reservoir 370 on the one or more surfaces of the gas turbine engine 10 including the foamed wash fluid 376. For example, the additive material may be injected into the gas turbine engine 10 after the foamed wash fluid 376. In other example embodiments, the foamed wash fluid 376 and the additive material 380 may be combined within the at least one supply line 378 or within a mixing chamber downstream of the foam wash module 315 and the additive reservoir 370 for simultaneously supplying the foamed wash fluid and the additive material to surfaces of the one or more components of the gas turbine engine 10.

The foamed wash fluid is configured to break up and remove dirt, debris, and other foreign materials present within the gas turbine engine 10. The foamed wash fluid may wet or soak (e.g., wash, scrub) the gas turbine engine 10 to facilitate removal of dirt, debris, and foreign materials disposed in the gas turbine engine 10. For example, the foreign materials may include pollutants (e.g., sulfate, nitrates, etc.), evaporate deposits (e.g., halite, carbonates, etc.), and dust (e.g., aluminosilicate clays), and the foamed cleaning material may include a particular composition suitable for removal of the pollutants, evaporate deposits, and/or dust.

Moreover, the additive material 380 reacts with the foamed wash fluid 376 to improve cleaning efficiency and provide cleaning in regions of the gas turbine engine 10 that the foamed wash fluid 376 may not otherwise reach without the additive material 380. In at least one example embodiment, the method 700 may additionally include maintaining a desired temperature of the foamed wash fluid 376 with the exothermic material. Additionally, or alternatively, the method 700 may include circulating the foamed wash fluid 376 about surfaces of the one or more components of the gas turbine engine 10 with the self-propelling material.

After cleaning, the method 700 may further include discharging the used foamed wash fluid from the gas turbine engine 10 along within any foreign particles and excess additive material to the collection module 320, as discussed above with respect to FIGS. 3.

Utilizing a wash system in accordance with one or more of the exemplary embodiments described herein may allow for more efficient cleaning of the gas turbine engine. More specifically, introducing additive material to foamed wash fluid within the gas turbine engine may provide improved cleaning and reduced overall cleaning time. The additive material may include exothermic materials for locally increasing a temperature of the foamed wash fluid for maintaining a desired temperature of the foamed wash fluid. Additionally, or alternatively, the additive material may include self-propelling materials for circulating and propelling the foamed wash fluid along surfaces of the gas turbine engine such that the foamed wash fluid can reach recesses, crevices, occluded surfaces, passageways, and other regions of the gas turbine engine that the foamed wash fluid may not otherwise be able to reach. Accordingly, the wash system of the present disclosure provides improved cleaning efficiency and reduced cleaning duration.

Further aspects are provided by the subject matter of the following clauses:
A wash system for washing a gas turbine engine, the gas turbine engine comprising a compressor section, a combustion section, and a turbine section, the wash system comprising: a detergent reservoir for storing a wash fluid; a foam wash module including a foam generating device in fluid communication with the detergent reservoir and configured to generate a flow of foamed wash fluid; one or more additive reservoirs for storing at least one additive material, the at least one additive material comprising a self-propelling material, an exothermic material, or both the self-propelling material and the exothermic material; and at least one supply line in communication with the foam wash module and the one or more additive reservoirs to supply the foamed wash fluid and the at least one additive material to the gas turbine engine.

The wash system of any preceding clause, wherein the additive material comprises the exothermic material, and the exothermic material is configured to maintain a desired temperature of the foamed wash fluid.

The wash system of any preceding clause, wherein the desired temperature of the foamed wash fluid is greater than or equal to 70°C and less than or equal to 90°C.

The wash system of any preceding clause, wherein the additive material comprises the exothermic material, and the exothermic material comprises nitrogen dioxide (NO2) and dinitrogen tetroxide (N2O4).

The wash system of any preceding clause, wherein the additive material comprises the exothermic material, and the exothermic material comprises ethylene oxide.

The wash system of any preceding clause, wherein the additive material comprises the self-propelling material, and the self-propelling material comprises a plurality of microtubules, each of the plurality of microtubules including at least one protein and an energy source.

The wash system of any preceding clause, wherein: the at least one protein comprises kinesin; and the energy source comprises adenosine triphosphate (ATP), gluten, enzymes, or a combination thereof.

The wash system of any preceding clause, wherein each of the plurality of microtubules comprise greater than or equal to 10% and less than or equal to 20% of the adenosine triphosphate (ATP).

The wash system of any preceding clause, wherein each of the plurality of microtubules comprise: a length less than or equal to 50 micrometers; and a width extending perpendicular to the length, the width greater than or equal to 23 nm and less than or equal to 27 nm.

The wash system of any preceding clause, wherein each of the plurality of microtubules comprise a cylindrical shape and define an inner diameter greater than or equal to 11 nm and less than or equal to 15 nm.

The wash system of any preceding clause, wherein the self-propelling material comprises organic materials, the organic material comprising proteins including kinesin, myosin, and dynein; gluten; enzymes; and cytoplasm found in eukaryotic organisms and algae.

A method for cleaning a gas turbine engine, the method comprising: coupling a wash system to at least one inlet of the gas turbine engine; aerating a flow of wash fluid to generate a flow of foamed wash fluid; depositing the foamed wash fluid on one or more components of the gas turbine engine; and depositing an additive material on the one or more components of the gas turbine engine including the foamed wash fluid, the additive material comprising a self-propelling material, an exothermic material, or both the self-propelling material and the exothermic material.

The method of any preceding clause, wherein the method comprises depositing the foamed wash fluid on the one or more components of the gas turbine engine before depositing the additive material on the one or more components of the gas turbine engine.

The method of any preceding clause, wherein depositing the foamed wash fluid on the one or more components of the gas turbine engine and depositing the additive material on the one or more components of the gas turbine engine occurs simultaneously.

The method of any preceding clause, wherein the additive material comprises the exothermic material, and wherein the method further comprises maintaining a desired temperature of the foamed wash fluid with the exothermic material.

The method of any preceding clause, wherein the desired temperature of the foamed wash fluid is greater than or equal to 70°C and less than or equal to 90°C.

The method of any preceding clause, wherein the additive material comprises the exothermic material, and the exothermic material comprises nitrogen dioxide (NO2) and dinitrogen tetroxide (N2O4).

The method of any preceding clause, wherein the additive material comprises the exothermic material, and the exothermic material comprises ethylene oxide.

The method of any preceding clause, wherein the additive material comprises the self-propelling material, and the method further comprises circulating the foamed wash fluid about surfaces of the one or more components of the gas turbine engine with the self-propelling material.

The method of any preceding clause, wherein the additive material comprises the self-propelling material, and the self-propelling material comprises a plurality of microtubules, each of the plurality of microtubules including at least one protein and an energy source.

The method of any preceding clause, wherein: the at least one protein comprises kinesin; and the energy source comprises adenosine triphosphate (ATP), gluten, enzymes, or a combination thereof.

The method of any preceding clause, wherein the plurality of microtubules comprise greater than or equal to 10% and less than or equal to 20% of the adenosine triphosphate (ATP).

The method of any preceding clause, wherein the self-propelling material comprises organic materials, the organic material comprising proteins including kinesin, myosin, and dynein; gluten; enzymes; and cytoplasm found in eukaryotic organisms and algae.

The method of any preceding clause, wherein the wash system comprises a plurality of wash lines, and wherein coupling the wash system to the at least one inlet of the gas turbine engine comprises coupling the plurality of wash lines to the at least one inlet of the gas turbine engine.

The method of any preceding clause, wherein the at least one inlet of the gas turbine engine is in fluid communication with one or more components of the gas turbine engine, the one or more components of the gas turbine engine comprising a compressor section, a combustion section, and a turbine section.

The method of any preceding clause, wherein: the depositing the foamed wash fluid comprises injecting the foamed wash fluid into the gas turbine engine at one or more locations; and the depositing the additive material comprises injecting the additive material into the gas turbine engine at one or more locations.

The method of any preceding clause, wherein the one or more locations of the gas turbine engine comprise one or more injection ports.

The method of any preceding clause, wherein the one or more injection ports comprise borescope ports disposed in one or more of a compressor section, a combustion section, and a turbine section of the gas turbine engine.

This written description uses examples to disclose the present disclosure, including the best mode, and also to enable any person skilled in the art to practice the disclosure, including making and using any devices or systems and performing any incorporated methods. The patentable scope of the disclosure is defined by the claims, and may include other examples that occur to those skilled in the art. Such other examples are intended to be within the scope of the claims if they include structural elements that do not differ from the literal language of the claims, or if they include equivalent structural elements with insubstantial differences from the literal languages of the claims.

## Claims

1. A wash system (300) for washing a gas turbine engine (10), the gas turbine engine (10) comprising a compressor section (22, 24), a combustion section (26), and a turbine section (28, 30), the wash system (300) comprising:
a detergent reservoir (365) for storing a wash fluid (372);
a foam wash module (315) including a foam generating device in fluid communication with the detergent reservoir (365) and configured to generate a flow of foamed wash fluid (376);
one or more additive reservoirs (370) for storing at least one additive material (380), the at least one additive material (380) comprising a self-propelling material, an exothermic material, or both the self-propelling material and the exothermic material; and
at least one supply line (378) in communication with the foam wash module (315) and the one or more additive reservoirs (370) to supply the foamed wash fluid (376) and the at least one additive material (380) to the gas turbine engine (10).

2. The wash system (300) of claim 1, wherein:
the additive material (380) comprises the exothermic material, and the exothermic material is configured to maintain a desired temperature of the foamed wash fluid (376); and
the desired temperature of the foamed wash fluid (376) is greater than or equal to 70°C and less than or equal to 90°C.

3. The wash system (300) of any one of the preceding claims, wherein the additive material (380) comprises the exothermic material, and the exothermic material comprises nitrogen dioxide (NO2) and dinitrogen tetroxide (N2O4) or ethylene oxide.

4. The wash system (300) of any one of the preceding claims, wherein:
the additive material (380) comprises the self-propelling material, and the self-propelling material comprises a plurality of microtubules (400), each of the plurality of microtubules (400) including at least one protein and an energy source
the at least one protein comprises kinesin; and
the energy source comprises adenosine triphosphate (ATP), gluten, enzymes, or a combination thereof.

5. The wash system (300) of claim 4, wherein each of the plurality of microtubules (400) comprise greater than or equal to 10% and less than or equal to 20% of the adenosine triphosphate (ATP).

6. The wash system (300) of any one of the preceding claims, wherein the self-propelling material comprises organic materials, the organic material comprising proteins including kinesin, myosin, and dynein; gluten; enzymes; and cytoplasm found in eukaryotic organisms and algae.

7. A method (700) for cleaning a gas turbine engine (10), the method (700) comprising:
coupling a wash system (300) to at least one inlet of the gas turbine engine (10);
aerating a flow of wash fluid (372) to generate a flow of foamed wash fluid (376);
depositing the foamed wash fluid (376) on one or more components of the gas turbine engine (10); and
depositing an additive material (380) on the one or more components of the gas turbine engine (10) including the foamed wash fluid (376), the additive material (380) comprising a self-propelling material, an exothermic material, or both the self-propelling material and the exothermic material.

8. The method (700) of claim 7, wherein the method (700) comprises depositing the foamed wash fluid (376) on the one or more components of the gas turbine engine (10) before depositing the additive material (380) on the one or more components of the gas turbine engine (10).

9. The method (700) of claim 7, wherein depositing the foamed wash fluid (376) on the one or more components of the gas turbine engine (10) and depositing the additive material (380) on the one or more components of the gas turbine engine (10) occurs simultaneously.

10. The method (700) of any one of claims 7-9, wherein:
the additive material (380) comprises the exothermic material, and wherein the method (700) further comprises maintaining a desired temperature of the foamed wash fluid (376) with the exothermic material; and
the desired temperature of the foamed wash fluid (376) is greater than or equal to 70°C and less than or equal to 90°C.

11. The method (700) of any one of claims 7-10, wherein the additive material (380) comprises the self-propelling material, and the method (700) further comprises circulating the foamed wash fluid (376) about surfaces of the one or more components of the gas turbine engine (10) with the self-propelling material.

12. The method (700) of any one of claims 7-11, wherein the wash system (300) comprises a plurality of wash lines (610), and wherein coupling the wash system (300) to the at least one inlet of the gas turbine engine (10) comprises coupling the plurality of wash lines (610) to the at least one inlet of the gas turbine engine (10).

13. The method (700) of any one of claims 7-12, wherein the at least one inlet of the gas turbine engine (10) is in fluid communication with one or more components of the gas turbine engine (10), the one or more components of the gas turbine engine (10) comprising a compressor section (22, 24), a combustion section (26), and a turbine section (28, 30).

14. The method (700) of any one of claims 7-13, wherein:
the depositing the foamed wash fluid (376) comprises injecting the foamed wash fluid (376) into the gas turbine engine (10) at one or more locations; and
the depositing the additive material (380) comprises injecting the additive material (380) into the gas turbine engine (10) at one or more locations.

15. The method (700) of claim 14, wherein the one or more locations of the gas turbine engine (10) comprise one or more injection ports, and wherein the one or more injection ports comprise borescope ports (620) disposed in one or more of a compressor section (22, 24), a combustion section (26), and a turbine section (28, 30) of the gas turbine engine (10).
